(19) Europäisches Patentamt
European Patent Office
Office européen des brevets

(11) **EP 4 151 728 A1**

(12) **EUROPEAN PATENT APPLICATION**
published in accordance with Art. 153(4) EPC

(43) Date of publication:
**22.03.2023 Bulletin 2023/12**

(21) Application number: **21803729.9**

(22) Date of filing: **14.05.2021**

(51) International Patent Classification (IPC):
**C12N 15/09** (2006.01)     **C12Q 1/6869** (2018.01)

(52) Cooperative Patent Classification (CPC):
**C12N 15/09; C12Q 1/6869; G16B 40/00**

(86) International application number:
**PCT/JP2021/018512**

(87) International publication number:
**WO 2021/230380 (18.11.2021 Gazette 2021/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.05.2020 JP 2020085433**

(71) Applicant: **Kao Corporation
Chuo-ku
Tokyo 103-8210 (JP)**

(72) Inventors:
• **UEHARA, Yuya
Haga-gun, Tochigi 321-3497 (JP)**
• **YAJIMA, Kotomi
Odawara-shi, Kanagawa 250-0002 (JP)**
• **INOUE, Takayoshi
Haga-gun, Tochigi 321-3497 (JP)**
• **OYA, Naoki
Odawara-shi, Kanagawa 250-0002 (JP)**

(74) Representative: **Vossius & Partner
Patentanwälte Rechtsanwälte mbB
Siebertstraße 3
81675 München (DE)**

(54) **METHOD FOR DATA PROCESSING OF RNA INFORMATION**

(57)     Provided is data processing of RNA information in order to perform effective normalization in the case of analyzing RNA information obtained from secretion derived from a subject.

A data processing method for analysis of RNA expression information obtained from secretion collected from a plurality of subjects as biological specimens, the method comprising the following steps a) to d):
a) a step of counting the number of detectable RNAs in detection target RNAs by judging RNAs of which the expression level is zero or can be regarded as zero to be undetectable, and determining proportion 1 (TD value) of the number of detectable RNAs with respect to the total number of the detection target RNAs for each specimen;
b) a step of excluding specimens for which proportion 1 is less than a threshold set within a range from 5% to 29% from the specimens to select analysis target specimens;
c) a step of determining, for each detection target RNA, proportion 2 (SD value) of the number of specimens for which the expression level thereof is higher than zero or higher than an expression level that can be regarded as zero with respect the total number of the analysis target specimens based on the RNA expression information of the analysis target specimens selected above; and
d) a step of excluding RNAs for which proportion 2 is less than a threshold set within a range from 81% to 99% from the detection target RNAs and extracting the expression information of RNAs other than the excluded RNAs as an analysis target.

EP 4 151 728 A1

**Description**

Field of the Invention

The present invention relates to a method for data processing of RNA information in a human-derived secretion.

Background of the Invention

In recent years, it has been developed techniques for examining human current and, furthermore, future in vivo physiological states by analysis of nucleic acids, such as DNAs and RNAs, in a biological specimen. The analysis using nucleic acids has advantages of being capable of obtaining abundant information by a single analysis because a comprehensive analysis method has been established and of being capable of easily performing functional association of analysis results based on many research reports on single nucleotide polymorphism, RNA function, and the like. Nucleic acids of a biological origin can be extracted from, for example, body fluid such as blood, secretion, or tissue, and recently, it has been reported that RNAs included in skin surface lipids (SSL) are used as a specimen for biological analysis and that marker genes of epidermis, sweat glands, hair follicles, and sebaceous glands are detected in SSL (Patent Literature 1).

RNA sequencing (RNA-Seq) analysis, which directly quantitatively measures the RNA sequence expressed in cells, can detect a low-expression gene of which quantitative measurement in a microarray using a signal intensity ratio is difficult, and can obtain a highly accurate expression profile, and is therefore an analysis approach that is currently attracting attention. In gene expression analysis, the concentration and/or relative or absolute amount of a specific RNA in a specimen is determined, and the specific RNA is quantified (quantitatively measured). In this case, a highly accurate and reproducible method is desired. However, in biological specimens collected from different individuals, since bias may occur in the expression level profile depending on the biological specimen or the analysis process, the quantities of a specific RNA are not always directly comparable. Accordingly, in biological specimens derived from two or more different individuals, in order to well compare the quantities of a specific RNA, normalization is performed for the quantities of the RNA across specimens.

In RNA-seq analysis, the number of sequence reads mapped on a genome is used for quantitative measurement of the expression level of a gene. Accordingly, the normalization uses, for example, RPM (Reads Per Million reads mapped, Non Patent Literature 1) or RLE (Relative Log Expression, Non Patent Literature 2), which are correction methods using the total number of reads. Normalization by RLE is implemented in an analytical technique for a series of gene expression level analysis called DESeq2.

However, information of RNAs collected from secretion such as sebum and saliva, in particular, RNAs collected from SSL, includes many missing values and is high in variation. Accordingly, when the same data processing as for information of other RNAs is performed, even if subsequent statistical processing, such as machine learning, is performed, problems may arise in accuracy and reproducibility.

[Patent Literature 1] WO 2018/008319

[Non Patent Literature 1] IPSJ SIG Technical Reports, Vol. 2013-BIO-33, No. 9, pp. 1-3
[Non Patent Literature 2] Genome Biol., 2014, Vol. 15, No. 12, p. 550

Summary of the Invention

The present invention relates to the following 1) to 3) .

1) A data processing method for analysis of RNA expression information obtained from secretion collected from a plurality of subjects as biological specimens, the method comprising the following steps a) to d) :

a) a step of counting the number of detectable RNAs in detection target RNAs by judging RNAs of which an expression level is zero or can be regarded as zero to be undetectable, and determining proportion 1 (TD value) of the number of detectable RNAs with respect to the total number of the detection target RNAs for each specimen;
b) a step of excluding specimens for which the proportion 1 is less than a threshold set within a range from 5% to 29% from the specimens to select analysis target specimens;
c) a step of determining, for each detection target RNA, proportion 2 (SD value) of the number of specimens in which the expression level thereof is higher than zero or higher than an expression level that can be regarded as zero with respect to the total number of the analysis target specimens based on the RNA expression information of the analysis target specimens selected above; and

d) a step of excluding RNAs for which the proportion 2 is less than a threshold set within a range from 81% to 99% from the detection target RNAs and extracting expression information of RNAs other than the excluded RNAs as an analysis target.

2) A method of correcting an RNA expression value, comprising normalizing the total RNA expression information extracted by the method of 1).

3) A program for implementing the data processing method of 1) or the correction method of 2), an information recording medium recording the program, a computing device implementing the program, and an RNA analysis data set obtained by the data processing method or the correction method.

Brief Description of Drawing

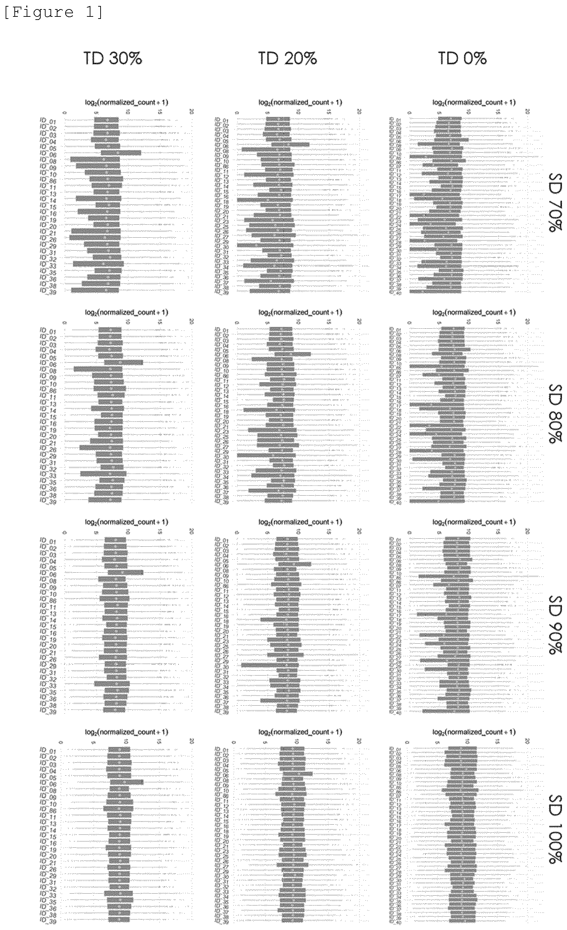

[Figure 1] Figure 1 is a box plot of $\text{Log}_2$ (normalized count + 1) value in each subject.

Detailed Description of the Invention

The present invention relates to a provision of a data processing method for RNA information in order to perform effective normalization processing in cases of using secretion derived from a subject as a biological specimen and analyzing the RNA information obtained therefrom.

The present inventors used the expression status of RNAs included in SSL as sequence information and examined the data used in normalization of expression values for various statistical approaches and, as a result, found that effective normalization processing is possible by setting a threshold as a selection criterion of data analysis target specimens and a threshold as a selection criterion of data analysis target genes within specific ranges and extracting RNA information.

According to the present invention, in biological specimens of which RNA expression information includes many missing values and variations, when RNA expression profiles derived from a plurality of samples are compared, effective normalization processing is possible, and statistical analysis with high accuracy and high reproducibility is possible based on the RNA information.

In the method of the present invention, the "RNA" as an analysis target may be any RNA of a biological origin and may be any of total RNA, mRNA, rRNA, tRNA, and noncoding RNA but is preferably mRNA.

The biological specimen used in the method of the present invention is secretion derived from a subject, and specifically, examples thereof include a specimen including sebum, saliva, runny nose, tears, sweat, urine, semen, vaginal fluid, amniotic fluid, milk, and feces. Among these specimens, the method of the present invention is effective to be applied to skin surface lipids (SSL) which includes many missing RNA information and is high in variation.

The term "skin surface lipids (SSL)" refers to a lipophilic fraction present on the surface of the skin and is also called sebum. In general, SSL mainly contains secretion secreted from exocrine glands, such as sebaceous glands, on the skin and is present on the skin surface in the form of a thin layer covering the skin surface. SSL contains RNAs expressed in skin cells. Here, the term "skin" is a generic name of regions including stratum corneum, epidermis, dermis, hair follicles, and tissues such as sweat glands, sebaceous glands, and other glands, unless otherwise specified.

In collection of SSL from the skin of a subject, any means used for collection or removal of SSL from the skin can be adopted. Preferably, an SSL absorbent material, an SSL adhesive material, or a tool for scraping off SSL from the skin can be used. The SSL absorbent material and the SSL adhesive material are not particularly limited as long as they have affinity to SSL, and examples thereof include polypropylene and pulp. More detailed examples of the procedure of collecting SSL from the skin include a method of absorbing SSL to a sheetlike material, such as oil-blotting paper and an oil-blotting film, a method of adhering SSL to a glass plate, tape, or the like, and a method of scraping off and collecting SSL with a spatula, scraper, or the like. In order to improve the adsorptive property of SSL, an SSL absorbent material impregnated with a solvent having high lipophilicity in advance may be used. In contrast, if the SSL absorbent material contains a solvent having high hydrophilicity, or moisture, adsorption of SSL is prevented. Accordingly, it is preferable that the content of a highly hydrophilic solvent or moisture be low. It is preferable to use the SSL absorbent material in a dry state. The site of the skin where SSL is collected is not particularly limited, and examples of the skin include those at any site of the body such as the head, face, neck, trunk, and limbs, and a site where sebum is abundantly secreted, for example, the skin of the head or the face is preferable, and the skin of the face is more preferable.

RNA-containing SSL collected from a subject may be stored for a certain period of time. The collected SSL is preferably stored at a low temperature condition as prompt as possible after collection in order to suppress the decomposition of RNAs contained therein as much as possible. The temperature condition for storing the RNA-containing SSL may be 0°C or less and is preferably from -20°C ± 20°C to -80°C ± 20°C, more preferably from -20°C ± 10°C to -80°C ± 10°C, further more preferably from -20°C ± 20°C to -40°C ± 20°C, further more preferably from -20°C ± 10°C to -40°C ± 10°C, further more preferably -20°C ± 10°C, and further more preferably -20°C ± 5°C. The period of time for storing the RNA-containing SSL at the low temperature condition is not particularly limited and is preferably 12 months or less, for example, 6 hours or more and 12 months or less, more preferably 6 months or less, for example, 1 day or more and 6 months or less, and further more preferably 3 months or less, for example, 3 days or more and 3 months or less.

In the method of the present invention, the method of acquiring RNA expression information is not particularly limited, and examples thereof include acquisition by converting RNAs included in a specimen into cDNAs by reverse transcription and then measuring the cDNAs or an amplification product thereof. Examples of the means of measuring an expression level include a DNA chip, a DNA microarray, and RNA-Seq, and the means is preferably RNA-Seq.

The RNA expression level of is quantitatively measured by a signal intensity ratio when microarray analysis is used and is quantitatively measured by the number of sequence reads (read count value) mapped on a genome in RNA-seq analysis.

The method of the present invention includes a step of acquiring information on the RNA expression level and includes a step of obtaining the number of sequence reads (read count value) quantitatively measured as the RNA expression level as described above. After the step, the data of the RNA expression level are stored in a server or a recording medium of a computer and input into a computer, and processing of the data of the present invention can be implemented by the program installed in the computer based on the input data.

In the data processing method of RNA information of the present invention, the expression information of analysis target RNA is extracted by setting a threshold as a selection criterion of data analysis target specimens and a threshold as a selection criterion of data analysis target genes, and normalization is performed.

As shown in Examples as described later, regarding the RNA expression level data (read count value by RNA-Seq) in a specimen derived from a subject, the selection criterion of specimens (subjects) as a data analysis target and the selection criterion of genes as a data analysis target were examined as follows. As a selection index of specimens (j) as a data analysis target, the $TD_j$ value determined for each specimen by the following equation is used. The TD value is Targets Detected and corresponds to the gene detection rate (%).

$$TD_j = \frac{number\ j\ of\ detectable\ genes}{total\ number\ of\ detection\ target\ genes} \times 100$$

Here, the total number of detection target genes is the total number of genes judged to be theoretically detectable in RNA expression analysis and may be appropriately determined based on the RNA expression analytical method to be used. In the case of the sequencing method (AmpliSeq) of Examples as described later, the total number is determined based on the number of primer pairs of Multiplex PCR.

In addition, the number of detectable genes can be calculated by subtracting the number of undetectable genes from the total number of detection target genes. Here, the number of undetectable genes means the number of genes of which the expression is zero or can be regarded as zero.

In selection of genes (i) as a data analysis target, the $SD_i$ value determined for each gene by the following equation is used. The SD value is Samples Detected and is a rate (detection specimen rate) of specimens for which the detection of expression of RNA derived from a gene has enabled, for each of the genes of the RNA expression level data of data analysis target specimens after selection using the TD value. Here, what the detection of expression of RNA has enabled means that the detection expression higher than zero or a level that can be regarded as zero has enabled.

$$SD_i = \frac{number\ _i\ of\ specimens\ (subjects)\ showing\ detectable\ RNA\ expression}{total\ number\ of\ analysis\ target\ specimens\ (subjects)} \times 100$$

Specimens (subjects) having a $TD_j$ value of 0% or less than 20% or 30% were excluded, and the other specimens (subjects) were selected as data analysis target specimens (subjects). Subsequently, genes having an $SD_i$ value of less than 70%, 80%, 90%, or 100% were excluded, and the other genes were selected as data analysis target genes. The RNA expression level data extracted for these genes were subjected to normalization by DESeq2 (Love MI, et al., Genome Biol., 2014) to verify the degree of approximation to a normal distribution. As a result, a possibility of better approximation to a normal distribution in normalization by DESeq2 was demonstrated by excluding specimens having a TD value of 0%, less than 20%, or less than 30% and excluding genes having an SD value of less than 80%, less than 90%, or less than 100%.

However, in this case, it was demonstrated that although about 80% of the number of analysis target specimens can be secured as analyzable specimens when specimens having a TD value of less than 20% are excluded, the number of analyzable specimens is decreased to about 60% when specimens having a TD value of less than 30% are excluded. It was also demonstrated that although the number of analyzable genes is less than 20% of the analysis target genes when genes having an SD value of less than 90% are excluded, the number is decreased to several percent when genes having an SD value of less than 100% are excluded.

Accordingly, in the present invention, RNAs of which the expression level is zero or can be regarded as zero are judged as undetectable, the number of detectable RNAs is counted, proportion 1 (TD value) of the number of detectable RNAs with respect to the total number of the detection target RNAs is determined for each specimen (step a), specimens for which the proportion 1 is less than a threshold set in a range from 5% to 29% are excluded to select analysis target specimens (step b), proportion 2 (SD value) of the number of specimens for which the RNA expression level is higher than zero or higher than an expression level that can be regarded as zero with respect to the total number of the analysis target specimens is determined for each detection target RNA in the selected specimens (step c), RNAs for which the proportion 2 is less than a threshold set in a range from 81% to 99% are excluded, and the other RNAs are used as analysis targets to extract the expression information thereof (step d). It is said that consequently, effective normalization is possible in subsequent normalization processing.

In the step a, the RNAs of which the expression level is zero or can be regarded as zero can be appropriately determined by a measurement means. For example, in RNA-seq analysis, such RNAs are those having a read count value of less than 20, preferably less than 15, and more preferably less than 10.

In the selection of analysis target specimens in the step b, the threshold of the proportion 1 of the number of detectable RNAs with respect to the total number of detection target RNAs is set to 5% or more from the viewpoint of effective normalization and is preferably 10% or more, more preferably 15% or more, and further more preferably 18% or more. At the same time, the threshold of the proportion 1 is set to 29% or less from the point of securing the number of analysis target specimens for analysis after the normalization and is preferably 27% or less, more preferably 25% or less, and further more preferably 23% or less. In addition, the threshold of the proportion 1 is appropriately set within a range from 5% to 29%, preferably within a range from 10% to 27%, more preferably within a range from 15% to 25%, and further more preferably within a range from 18% to 23%. The threshold of the proportion 1 is particularly preferably set to 20%.

In the step c, for each detection target RNA, the proportion 2 (SD value) of the number of specimens for which the expression level is higher than zero or higher than an expression level that can be regarded as zero with respect to the total number of the analysis target specimens is calculated. Here, the expression level that can be regarded as zero means that, for example, in RNA-seq analysis, the read count value is less than 5, preferably less than 3, and more preferably less than 1. In the present invention, as the proportion 2 (SD value), proportion of the number of specimens for which the expression level is higher than zero (in RNA-seq analysis, the number of specimens having a read count value of higher than 0) with respect to the total number of analysis target specimens is preferably used.

In addition, in the selection of analysis target RNAs in the step d, the threshold of the proportion 2 of the number of specimens for which the RNA expression level is higher than zero or higher than an expression level that can be regarded as zero with respect to the total number of the specimens is set to 81% or more from the viewpoint of effective normalization and is preferably 84% or more and more preferably 87% or more. At the same time, the threshold of the proportion 2 is set to 99% or less from the point of securing the number of analysis target genes for analysis after the normalization and is preferably 96% or less and more preferably 93% or less. In addition, the threshold of the proportion 2 is appropriately set within a range from 81% to 99%, preferably within a range from 84% to 96% and more preferably within a range from 87% to 93%. The threshold of the proportion 2 is particularly preferably set to 90%.

When the threshold of the proportion 1 in the step b is low, it is desirable that the threshold of the proportion 2 in the step d be set to be high for efficient normalization. When the threshold of the proportion 2 in the step d is low, it is desirable that the threshold of the proportion 1 in the step b be set to be high for efficient normalization.

Thus, effective correction of RNA expression values approximated to a normal distribution is possible by normalization the total extracted expression information of analysis target RNAs.

The method for normalization used in this case is not particularly limited, and, for example, in addition to the above-described RPM method and RLE method, FPKM (fragments per kilobase of exon per million reads mapped) method, RPKM (reads per kilobase of exon per million reads mapped), TPM (transcripts per million) method, TMM (Trimmed mean of M values) method, or the like can be adopted, and the RLE method is suitably used. The RLE method is implemented in an analytical method for performing a series of gene expression level analysis called DESeq2.

The data processing method and correction method for analysis of the RNA expression information can be performed using a computer (computing device). That is, the present invention can provide a computing device for implementing the above method, a program for implementing the method by the computer, and a computer-readable information recording medium on which the program is recorded. Furthermore, the present invention can provide a data set for RNA analysis obtained by the above data processing method. In addition, the present invention can perform data processing by inputting information, such as the proportion 1, the proportion 2, or the threshold, used for the above data processing or can also select the proper proportion 1, proportion 2, and thresholds by computation.

The computing device of the present invention includes a means for inputting RNA expression information obtained from a specimen collected from a subject and, includes one or more steps selected from the group consisting of the above-described steps of selecting analysis target specimens, selecting analysis target genes, extracting RNA expression information of the analysis target genes, and normalization of the RNA expression information according to the program for implementing the data processing method and correction method of the present invention.

Examples of the computer-readable information recording medium that records the program for implementing the data processing method and correction method of the present invention include a magnetic disk, an optical disk, a magneto-optical disk, and a flash memory. In the present invention, the term computer-readable includes the case of distribution via an electric communication line or the like.

Aspects and preferable embodiments of the present invention are shown below.

<1> A data processing method for analysis of RNA expression information obtained from secretion collected from a plurality of subjects as biological specimens, the method comprising the following steps a) to d):

a) a step of counting the number of detectable RNAs in detection target RNAs by judging RNAs of which an expression level is zero or can be regarded as zero to be undetectable, and determining proportion 1 (TD value) of the number of detectable RNAs with respect to the total number of the detection target RNAs for each specimen;
b) a step of excluding specimens for which the proportion 1 is less than a threshold set within a range from 5% to 29% from the specimens to select analysis target specimens;
c) a step of determining, for each detection target RNA proportion 2 (SD value) of the number of specimens for which the expression level thereof is higher than zero or higher than an expression level that can be regarded as zero with respect to the total number of the analysis target specimens based on the RNA expression information of the analysis target specimens selected above; and
d) a step of excluding RNAs for which the proportion 2 is less than a threshold set within a range from 81% to 99% from the detection target RNAs and extracting expression information of RNAs other than the excluded RNAs as an analysis target.

<2> The method according to <1>, wherein the secretion is skin surface lipids.
<3> The method according to <1> or <2>, wherein the information on the RNA expression level in the step a) is a read count value by RNA-Seq.
<4> The method according to any one of <1> to <3>, wherein the RNAs of which the expression level is zero or can be regarded as zero in the step a) are RNAs of which the read count value by RNA-seq is less than 20, preferably less than 15, and more preferably less than 10.
<5> The method according to any one of <1> to <4>, wherein the threshold of the proportion 1 in the step b) is set to preferably 10% or more, more preferably 15% or more, and further more preferably 18% or more; and preferably 27% or less, more preferably 25% or less, and further more preferably 23% or less; or is set preferably within a range from 10% to 27%, more preferably within a range from 15% to 25%, and further more preferably within a range from 18% to 23%.
<6> The method according to any one of <1> to <4>, wherein the threshold of the proportion 1 in the step b) is set to 20%.
<7> The method according to any one of <1> to <6>, wherein the expression level that can be regarded as zero in the step c) is a read count value in RNA-seq of less than 5, preferably less than 3, and more preferably less than 1.
<8> The method according to any one of <1> to <6>, wherein the specimens for which the expression level is higher than zero or higher than an expression level that can be regarded as zero in the step c) are specimens for which the read count value in RNA-seq is higher than 0.
<9> The method according to any one of <1> to <8>, wherein the threshold of the proportion 2 in the step d) is set to preferably 84% or more and more preferably 87% or more; and preferably 96% or less and more preferably 93% or less; or is set preferably within a range from 84% to 96% and more preferably within a range from 87% to 93%.
<10> The method according to any one of <1> to <8>, wherein the threshold of the proportion 2 in the step d) is set to 90%.
<11> A method of correcting an RNA expression value, comprising normalizing the total RNA expression information extracted by the method according to any one of <1> to <10>.
<12> The method according to <11>, wherein the normalization is performed by an RLE method.
<13> A program for implementing the data processing method or correction method according to any one of <1> to <12> for analysis of RNA expression information.

<14> An information recording medium which records the program according to <13>.

<15> A computing device comprising one or more steps selected from the group consisting of a step of selecting analysis target specimens, a step of selecting analysis target genes, a step of extracting RNA expression information of the analysis target genes, and a step of calculating normalization of the RNA information of the analysis target genes that are implemented by the program according to <13>.

<16> An RNA analysis data set obtained by the data processing method or correction method according to any one of <1> to <12> for analysis of RNA expression information.

Examples

The present invention will now be described in further detail based on Examples but is not limited thereto.

Example 1: Normalization of RNA expression data extracted from SSL

1) SSL collection

Sebum was collected from the entire face of each of 42 healthy subjects (females aged 20 to 59) using an oil-blotting film, and the oil-blotting film was then transferred in a vial and was stored at -80°C for about one month until to be used for RNA extraction.

2) RNA preparation and sequencing

The oil-blotting films of the above 1) were each cut into a suitable size, and RNAs were extracted using QIAzol Lysis Reagent (Qiagen) in accordance with the attached protocol. The extracted RNAs were reverse transcribed at 42°C for 90 minutes using a SuperScript VILO cDNA Synthesis kit (Life Technologies Japan Ltd.) to synthesize cDNAs. As the primers of the reverse transcription reaction, random primers attached to the kit were used. A library containing DNAs derived from 20,802 genes was prepared from the resulting cDNAs by multiplex PCR. The multiplex PCR was performed using Ion AmpliSeq Transcriptome Human Gene Expression Kit (Life Technologies Japan Ltd.) under conditions of [99°C, 2 min → (99°C, 15 sec → 62°C, 16 min) × 20 cycles → 4°C, Hold] . The resulting PCR product was purified with Ampure XP (Beckman Coulter, Inc.) and was then subjected to buffer reconstruction, digestion of primer sequences, adapter ligation, purification, and amplification to prepare a library. The prepared library was loaded on Ion 540 Chip and sequenced using Ion S5/XL system (Life Technologies Japan Ltd.).

3) Data analysis

In the RNA expression level data (read count value) derived from the subjects measured in the above 2), a selection criterion of data analysis target subjects and a selection criterion of data analysis target genes were examined. As the selection criterion of data analysis target subjects, the value of Targets Detected (TD) calculated in Torrent Suite (Life Technologies Japan Ltd.) was used. The threshold of $TD_j$ calculated for each subject was set to 0%, 20%, and 30%, subjects of less than a threshold were excluded from the analysis target, and the other subjects were selected as data analysis target subjects. As an extraction criterion of data analysis target genes, percentage (Samples Detected, SD) of the subjects having a read count value of higher than 0 was used for each gene of RNA expression level data after the selection of data analysis target subjects using TD. The threshold of $SD_i$ calculated for each detection target gene was set to 70%, 80%, 90%, and 100%, genes of less than a threshold were excluded from the analysis target, and the other genes were selected as data analysis target genes. Data analysis target subjects were selected, subsequently, the expression information on the selected data analysis target genes was extracted, and logarithmic value (Log2 (normalized count + 1) value) to base 2 of a value obtained by adding integer 1 to the read count value (normalized count value) normalized using a method of DESeq2 was then calculated. Figure 1 shows a box plot of Log2 (normalized count + 1) value in each subject.

Here, the values of $TD_j$ of subject j (j: an integer from 1 to n, n: the number of subjects) and $SD_i$ of gene i (i: an integer from 1 to m, m: the number of detection target genes) were calculated as follows.

$$TD_j = \frac{\text{total number of detection target genes} - \text{number}_j \text{ of genes having a read count value of less than 10}}{\text{total number of detection target genes}} \times 100$$

$$SD_i = \frac{number_i\ of\ subjects\ having\ a\ read\ count\ value\ of\ higher\ than\ 0}{total\ number\ of\ analysis\ target\ subjects} \times 100$$

4) Setting of optimum selection criterion

The variance of the median value was calculated for the Log2 (normalized count + 1) value calculated in the above 3), and as a result, the variance of the median value was decreased to 0.1 or less with an increase in the threshold of the TD value or SD value (Table 1, boldface). A synergistic decrease in the variance of the median value with an increase in the threshold of the TD value or SD value was also confirmed. Accordingly, it was demonstrated that the median value of each subject after the normalization by DESeq2 can be adjusted by selection of data analysis target subjects and data analysis target genes using the TD value and the SD value. However, when subjects for which the TD value was less than 20% were excluded, the proportion of analyzable subjects was decreased to about 83%, but when subjects for which the TD value is less than 30% were excluded, the proportion of analyzable subjects was decreased to about 64% (Table 2). It was demonstrated that since it is necessary to secure the number of analysis target subjects in analysis after normalization, it is suitable to set the threshold for selection of data analysis target subjects to a TD value of 20% (Table 2, boldface). When genes for which SD value is less than 90% were excluded, the proportion of analyzable genes was about 16%, but when genes for which the SD value was less than 100% were excluded, the proportion of analyzable genes was decreased to 2% or 6% (Table 3). It was demonstrated that since it is necessary to secure the number of analysis target genes in analysis after normalization, it is suitable to set the threshold for selection of data analysis target genes to an SD value of 90% (Table 3, boldface).

[Table 1]

| Median variance | SD 70% | SD 80% | SD 90% | SD 100% |
|---|---|---|---|---|
| TD 0% | 2.39 | 1.50 | 0.49 | **0.041** |
| TD 20% | 0.66 | 0.18 | **0.041** | **0.033** |
| TD 30% | 0.17 | **0.10** | **0.047** | **0.024** |

[Table 2]

| Number of subjects (proportion) | SD 70% | SD 80% | SD 90% | SD 100% |
|---|---|---|---|---|
| TD 0% | (NA) | (NA) | (NA) | **42 (100%)** |
| TD 20% | (NA) | (NA) | **35 (83%)** | **35 (83%)** |
| TD 30% | (NA) | 27 (64%) | 27 (64%) | 27 (64%) |
| NA; not applicable (out of the target)) | | | | |

[Table 3]

| Number of genes (proportion) | SD 70% | SD 80% | SD 90% | SD 100% |
|---|---|---|---|---|
| TD 0% | (NA) | (NA) | (NA) | 451 (2%) |

(continued)

| Number of genes (proportion) | SD 70% | SD 80% | SD 90% | SD 100% |
|---|---|---|---|---|
| TD 20% | *(NA)* | *(NA)* | **3282 (16%)** | 1151 (6%) |
| TD 30% | *(NA)* | *(NA)* | *(NA)* | *(NA)* |
| NA; not applicable (out of the target) | | | | |

**Claims**

1. A data processing method for analysis of RNA expression information obtained from secretion collected from a plurality of subjects as biological specimens, the method comprising the following steps a) to d):

   a) a step of counting the number of detectable RNAs in detection target RNAs by judging RNAs of which an expression level is zero or can be regarded as zero to be undetectable, and determining proportion 1 (TD value) of the number of detectable RNAs with respect to the total number of the detection target RNAs for each specimen;
   b) a step of excluding specimens for which proportion 1 is less than a threshold set within a range from 5% to 29% from the specimens to select analysis target specimens;
   c) a step of determining, for each detection target RNA, proportion 2 (SD value) of the number of specimens for which the expression level thereof is higher than zero or higher than an expression level that can be regarded as zero with respect to the total number of the analysis target specimens based on the RNA expression information of the analysis target specimens selected above; and
   d) a step of excluding RNAs for which the proportion 2 is less than a threshold set within a range from 81% to 99% from the detection target RNAs and extracting expression information of RNAs other than the excluded RNAs as an analysis target.

2. The method according to Claim 1, wherein the secretion is skin surface lipids.

3. The method according to Claim 1 or 2, wherein the information on the RNA expression level in the step a) is a read count value by RNA-Seq.

4. The method according to any one of Claims 1 to 3, wherein the RNAs of which the expression level is zero or can be regarded as zero in the step a) are RNAs of which the read count value by RNA-seq is less than 10.

5. The method according to any one of Claims 1 to 4, wherein the threshold of the proportion 1 in the step b) is set to 20%.

6. The method according to any one of Claims 1 to 5, wherein the specimens for which the expression level is higher than zero or higher than an expression level that can be regarded as zero in the step c) are specimens for which the read count value in RNA-seq is higher than 0.

7. The method according to any one of Claims 1 to 6, wherein the threshold of the proportion 2 in the step d) is set to 90%.

8. A method of correcting an RNA expression value, comprising normalizing the total RNA expression information extracted by the method according to any one of Claims 1 to 7.

9. A program for implementing the data processing method or correction method according to any one of Claims 1 to 8 for analysis of RNA expression information.

10. An information recording medium which records the program according to Claim 9.

11. A computing device comprising one or more steps selected from the group consisting of a step of selecting analysis target specimens, a step of selecting analysis target genes, a step of extracting RNA expression information of the analysis target genes, and a step of calculating normalization of the RNA information of the analysis target genes that are implemented by the program according to Claim 9.

12. An RNA analysis data set obtained by the data processing method according to any one of Claims 1 to 8 for analysis of RNA expression information.

EP 4 151 728 A1

[Figure 1]

| INTERNATIONAL SEARCH REPORT | International application No. |
| | PCT/JP2021/018512 |

**A. CLASSIFICATION OF SUBJECT MATTER**
C12N 15/09(2006.01)i; C12Q 1/6869(2018.01)i
FI: C12Q1/6869 Z; C12N15/09 Z
According to International Patent Classification (IPC) or to both national classification and IPC

**B. FIELDS SEARCHED**

Minimum documentation searched (classification system followed by classification symbols)
C12N15/00-15/90; C12Q1/00-3/00; G16B5/00-99/00

Documentation searched other than minimum documentation to the extent that such documents are included in the fields searched
| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII), CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | LOVE, Michael I. et al, "Moderated estimation of fold change and dispersion for RNA-seq data with DESeq2", Genome Biol., 2014, vol. 15; 550, pp. 1-21 abstract, page 2, left column, line 30 to right column, line 13, page 14, left column, lines 13-32 | 9-11<br>1-8 |
| X<br>A | COLE, Michael B. et al., "Performance Assessment and Selection of Normalization Procedures for Single-Cell RNA-Seq", Cell Syst., 2019, vol. 8, pp. 315-328, e1-e8, Supplemental Information abstract, page 318, left column, lines 25-28, page e1, lines 23-42, page e2, lines 16-23, fig. 1F | 9-11<br>1-8 |
| X<br>A | WO 2018/229487 A1 (ONCOLOGICA UK LTD.) 20 December 2018 (2018-12-20) page 20, line 14 to page 21, line 10 | 9-11<br>1-8 |
| A | WO 2020/091044 A1 (KAO CORP.) 07 May 2020 (2020-05-07) claim 1, test examples 6-7 | 1-8 |

☐ Further documents are listed in the continuation of Box C.　　☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 July 2021 (09.07.2021) | 20 July 2021 (20.07.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

## INTERNATIONAL SEARCH REPORT

International application No.

PCT/JP2021/018512

**Box No. II**      **Observations where certain claims were found unsearchable (Continuation of item 2 of first sheet)**

This international search report has not been established in respect of certain claims under Article 17(2)(a) for the following reasons:

1. ☒ Claims Nos.: 12

because they relate to subject matter not required to be searched by this Authority, namely:

      Claim 12, which pertains to a "data set for RNA analysis", simply presents information, and thus relates to a subject matter on which this International Searching Authority is not required to carry out a search under the provisions of PCT Article 17(2)(a)(i) and PCT Rule 39.1(iv).

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

**Box No. III**      **Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

| International application No. |
|---|
| PCT/JP2021/018512 |

| Patent Documents referred in the Report | Publication Date | Patent Family | Publication Date |
|---|---|---|---|
| WO 2018/229487 A1 | 20 Dec. 2018 | JP 2020-523031 A<br>US 2020/0140957 A1<br>EP 3638815 A1<br>CA 3066905 A<br>AU 2018284125 A<br>CN 111051535 A<br>SG 11201912011Y A | |
| WO 2020/091044 A1 | 07 May 2020 | JP 2020-74769 A | |

Form PCT/ISA/210 (patent family annex) (January 2015)

## REFERENCES CITED IN THE DESCRIPTION

**Non-patent literature cited in the description**

- *IPSJ SIG Technical Reports,* (9), 1-3
- *Genome Biol.,* 2014, vol. 15 (12), 550
- **LOVE MI et al.** *Genome Biol.,* 2014